# EUROPEAN PATENT SPECIFICATION

(11) **EP 3 842 048 B1**
(45) Date of publication and mention of the grant of the patent: **02.10.2024**
(21) Application number: 18931027.9
(22) Date of filing: 20.08.2018
(51) Int. Cl.: A61K 31/737, A61K 9/19, A61P 3/00, A61P 13/10, A61P 19/02, A61K 9/00, A61K 47/02

(54) **NOVEL PENTOSAN POLYSULFATE SODIUM PREPARATION**
NEUARTIGES PENTOSANPOLYSULFATPRÄPARAT
NOUVELLE PRÉPARATION DE PENTOSANE POLYSULFATE SODIQUE

(43) Date of publication of application: 30.06.2021
(73) Proprietor: ReqMed Company, Ltd., Tokyo 194-0022 (JP)
(72) Inventor: MATSUMOTO Tadashi, Machida-shi, Tokyo 194-0003 (JP)
(74) Representative: J A Kemp LLP
(86) International application number: PCT/JP2018/030666
(87) International publication number: WO 2020/039480

(56) References cited:
- EP-A1- 3 683 242
- WO-A1-2017/131130
- WO-A1-2018/043668
- WO-A2-92/17214
- AU-B2- 2007 240 993
- JP-A- 2009 532 467
- JP-A- 2018 522 940
- JP-B1- 6 281 659
- US-A1- 2016 002 366
- NAKAMURA, TATSUFUMI: "Treatment Outcomes for HTLV-I Associated Myelopathy (HAM)", RINSHO SHINKEIGAKU, vol. 52, no. 12, 1 January 2012 (2012-01-01), pages 1524, XP055790658, DOI: 10.5692/clinicalneurol.52.1397
- KUMAGAI, KENJI ET AL.: "Clinical Study of Gonarthrosis Using Pentosan Sulfate", THE JOURNAL OF THE JAPANESE ORTHOPAEDIC ASSOCIATION, vol. 81, no. 4, 2007, pages S593, XP009526663, ISSN: 0021-5325

## Description

### Technical Field

The present invention relates to a lyophilized pentosan polysulfate preparation.

### Background Art

Pentosan polysulfate sodium is a semisynthetic compound obtained by chemically modifying polysaccharides extracted from European beeches, and was initially developed as an anticoagulant in Germany. Currently, a liquid injection (SP54 (registered trademark)) and an encapsulated oral preparation (Elmiron (registered trademark)) are marketed as therapeutic agents for interstitial cystitis. Moreover, pentosan polysulfate sodium has been known to have an effect of treating arthritis in animals (Non-Patent Document 1). A lyophilized pentosan polysulfate preparation has not been known in the past.

### Citation List

### Non-Patent Document

Non-Patent Document 1: Wijekoon et al., BMC veterinary Research (2018) 14: 152

### Patent Documents

EP3683242 relates to pentosan polysulfate having a uronic acid content of 0.0% by mass to 6.0% by mass.

WO92/17214 relates to physically and chemically stabilized polyatomic clusters for magnetic resonance image and spectral enhancement.

US2016/002366 relates to conjugate compounds of hyalauronic acid bonded to pentosan polysulfate which are shown to induce chondrogenic differentiation of a population of mesenchymal stem cells.

AU2007240993 relates to stabilized pentosan polysulfate formulations and methods of analyzing them.

### Summary of Invention

### Technical Problem

While developing a therapeutic agent for human arthritis by using a pentosan polysulfate injection, the inventors of the present invention found that minute particles were generated due to delamination in an ampule of a pentosan polysulfate injection for treatment of human arthritis. Although a pentosan polysulfate injection has been used over the past 30 or more years, such findings had not been obtained. Accordingly, the present invention was achieved in order to prevent the occurrence of delamination in a pentosan polysulfate injection.

### Solution to Problem

Based on the thought that using a lyophilized pentosan polysulfate sodium preparation makes it possible to prevent delamination in a pentosan polysulfate injection, the inventors of the present invention investigated various lyophilized preparations and various methods for manufacturing the lyophilized preparations. As a result, the inventors of the present invention succeeded in producing a lyophilized pentosan polysulfate preparation for the first time and found that delamination did not occur when the lyophilized preparation was reconstituted, and thus the present invention was completed.

### Description of Embodiments

The present invention relates to a lyophilized preparation for injection of pentosan polysulfate or a salt thereof, comprising pentosan polysulfate or a salt thereof, a buffer, and no cryoprotectants,
wherein the buffer is a phosphate buffer and comprises disodium hydrogen phosphate dodecahydrate in an amount such that the concentration is 1 to 4 mg/mL after reconstitution, and sodium dihydrogen phosphate dihydrate in an amount such that the concentration is 4.5 to 9 mg/mL after reconstitution, and
wherein the cryoprotectant is saccharide, and
wherein the salt of pentosan polysulfate is pentosan polysulfate sodium that is present in an amount such that the concentration is 80 to 120 mg/mL after reconstitution.
. In this specification, "pentosan polysulfate" is a polysaccharide that includes 1-4 linked β-D-xylanopyranose units as a basic skeleton and has a weight average molecular weight of 1000 to 6000 daltons or 1500 to 6000 daltons. For example, the pentosan polysulfate is represented by the formula below. 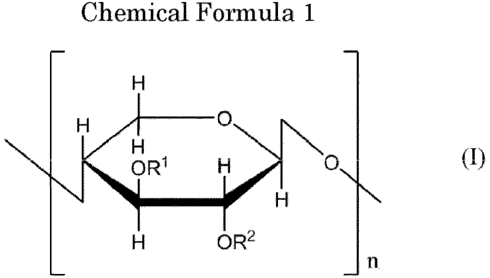 (In this formula, R¹ and R² represent SOsH.)

It is known that, in the pentosan polysulfate, one R² per *n* of about 3 to 15 may be a 1-4 linked acetyl-substituted β-D-xylanopyranose group represented by the formula below. Accordingly, the pentosan polysulfate sodium in this specification may include a polysaccharide represented by the formula above in which one R² per *n* of 3 to 15 (preferably 6 to 12 or 8 to 10) on average is a 1-4 linked acetyl-substituted β-D-xylanopyranose group represented by the formula below.

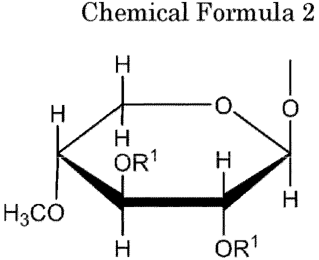

Pentosan polysulfate is described in Merck Index 11th Edition, Merck & Co, Inc., Rahway, N.J. (1989), p.7093; U.S. Patent No. 5180715 and U.S. Patent No. 5643892; and U.S. Publication No. 2001/0034328.

The pentosan polysulfate of the present invention includes sulfate groups and thus forms a salt with a base. Accordingly, the pentosan polysulfate salt of the present invention is the sodium salt of pentosan polysulfate. The term "pharmacologically acceptable salt" refers to a salt that is formed by the pentosan polysulfate of the present invention binding to an inorganic or organic base and is acceptable as a medicine to be administered to the body. Such salts are, for example, described in Berge et al., J. Pharm. Sci. 66: 1-19 (1977) and so on. Examples of further salts include salts formed with alkali metals and alkali earth metals such as zinc, lithium, sodium, potassium, magnesium, calcium, silver, lead, copper, gold, palladium, and barium; salts formed with amines such as ammonia, methylamine, dimethylamine, trimethylamine, dicyclohexylamine, tris(hydroxymethyl)aminomethane, N,N-bis(hydroxyethyl)piperazine, 2-amino-2-methyl-1-propanol, ethanolamine, N-methylglucamine, and L-glucamine; and salts with basic amino acids such as lysine, δ-hydroxylysine, and arginine. Specific examples thereof include pentosan polysulfate sodium, pentosan polysulfate calcium, and pentosan polysulfate potassium, wherein pentosan polysulfate sodium is the salt according to the invention.

The lyophilized preparation of the present invention contains pentosan polysulfate sodium (R¹ and R² represent SOsNa in Formula (I)) such that its concentration after reconstitution is 80 to 120 mg/mL, and preferably 90 to 110 mg/mL, 95 to 105 mg/mL, or 100 mg/mL.

The lyophilized preparation of the present invention contains a phosphate buffer comprising disodium hydrogen phosphate dodecahydrate and sodium dihydrogen phosphate dihydrate. In this specification, the "buffer" means a substance that serves to adjust a pH to be within a target value range when the lyophilized preparation of the present invention is reconstituted and that can be administered as a medicine to mammals. Examples of buffers include, but are not limited to, a borate buffer (containing sodium borate and sodium hydroxide), a phosphate buffer (containing sodium dihydrogen phosphate and disodium hydrogen phosphate), a carbonate-bicarbonate buffer (containing sodium carbonate and sodium hydrogen carbonate), a citrate buffer (containing sodium citrate and citric acid), an acetate buffer (containing acetic acid and sodium acetate), a succinate buffer, a histidine buffer, a tartrate buffer, HBSS, tris(hydroxymethyl)aminomethane (THAM), a citrate/phosphate buffer, a barbital buffer, a Britton-Robinson buffer, a cacodylate buffer, a collidine buffer, a formate buffer, a maleate buffer, a Mcllvaine buffer, a Prideaux-Ward buffer, a citratephosphate-borate buffer (Teorell-Stanhagen buffer), a veronal acetate buffer, a MES (2-(N-morpholino)ethanesulfonic acid) buffer, a BIS-TRIS (bis(2-hydroxyethyl)iminotris(hydroxymethyl)methane) buffer, an ADA (N-(2-acetamido)-2-iminodiacetic acid) buffer, an ACES (N-(carbamoylmethyl)-2-aminoethanesulfonic acid (sulfonaic acid)) buffer, a PIPES (piperazine-N, N'-bis(2-ethanesulfonic acid)) buffer, a MOPSO (3-(N-morpholino)-2-hydroxypropanesulfonic acid) buffer, a BIS-TRIS PROPANE (1,3-bis(tris(hydroxymethyl)methylamino) propane) buffer, a BES (N,N-bis(2-hydroxyethyl)-2-aminoethanesulfonic acid (sulfonaic acid)) buffer, a MOPS (3-(N-morpholino)propanesulfonic acid) buffer, a TES (N-tris(hydroxymethyl)methyl-2-aminoethanesulfonic acid) buffer, a HEPES (N-(2-hydroxyethyl)piperazine-N'-2-ethanesulfonic acid) buffer, a DIPSO (3-(N,N-bis(2-hydroxyethyl)amino)-2-hydroxypropanesulfonic acid) buffer, a MOBS (4-(N-morpholino)butanesulfonic acid) buffer, a TAPSO (3-(N-tris(hydroxymethyl)methylamino)-2-hydroxypropanesulfonic acid) buffer, a tris(hydroxymethylaminomethane) buffer, a HEPPSO (N-(2-hydroxyethyl)piperazine-N'-2-hydroxypropanesulfonic acid) buffer, a POPSO (piperazine-N,N'-bis(2-hydroxypropanesulfonic acid)) buffer, a TEA (triethanolamine) buffer, an EPPS (N-(2-hydroxyethyl)piperazine-N'-3-propanesulfonic acid) buffer, a TRICINE (N-tris(hydroxymethyl)methylglycine) buffer, a GLY-GLY (glycylglycine) buffer, a BICINE (N,N-bis(2-hydroxyethyl)glycine) buffer, a HEPBS (N-(2-hydroxyethyl)piperazine-N'-(4-butanesulfonic acid)) buffer, a TAPS (N-tris(hydroxymethyl)methyl-3-aminopropanesulfonic acid) buffer, and an AMPD (2-amino-2-methyl-1,3-propanediol) buffer. In the present invention the buffer is a phosphate buffer.

The lyophilized preparation of the present invention may contain a buffer such that the pH after reconstitution is 4.8 to 7.4, 5.0 to 7.2, 5.0 to 7.0, or 5.2 to 7.0.

The lyophilized preparation of this specification contains a pH adjuster as needed. Examples of the pH adjuster include potassium hydroxide, sodium hydroxide, lactic acid, hydrochloric acid, adipic acid, aqueous ammonia, dry sodium carbonate, diluted hydrochloric acid, a citric acid hydrate, a sodium citrate hydrate, sodium dihydrogen citrate, glycine, glucono-δ-lactone, gluconic acid, crystalline sodium dihydrogen phosphate, succinic acid, acetic acid, ammonium acetate, a sodium acetate hydrate, diisopropanolamine, tartaric acid, D-tartaric acid, L-sodium tartrate, calcium hydroxide, magnesium hydroxide, sodium hydrogen carbonate, a sodium carbonate hydrate, triisopropanolamine, triethanolamine, carbon dioxide, a calcium lactate hydrate, sodium lactate, glacial acetic acid, monosodium fumarate, sodium propionate, boric acid, ammonium borate, borax, maleic acid, anhydrous citric acid, anhydrous sodium acetate, anhydrous sodium monohydrogen phosphate, anhydrous sodium dihydrogen phosphate, meglumine, methanesulfonic acid, monoethanolamine, sulfuric acid, a potassium aluminum sulfate hydrate, DL-malic acid, phosphoric acid, trisodium phosphate, a sodium hydrogen phosphate hydrate, dipotassium phosphate, potassium dihydrogen phosphate, and sodium dihydrogen phosphate.

The lyophilized preparation of the present invention does not contain a saccharide cryoprotectant. In particular, common lyophilized preparations require a cryoprotectant such as a saccharide (e.g., maltose or mannitol) to maintain the stability, whereas the lyophilized preparation of this specification contains no cyroprotectants but can be provided as a stable preparation. Accordingly, the present invention includes a lyophilized preparation containing no saccharide cryoprotectants.

The lyophilized preparation of the present invention contains pentosan polysulfate sodium, disodium hydrogen phosphate dodecahydrate, and sodium dihydrogen phosphate dihydrate such that their concentrations after reconstitution are 80 to 120 mg/mL (preferably 90 to 110 mg/mL, 95 to 105 mg/mL, or 100 mg/mL), 1 to 4 mg/mL, and 4.5 to 9.0 mg/mL, respectively. More preferably, the lyophilized preparation of the present invention contains pentosan polysulfate sodium, disodium hydrogen phosphate dodecahydrate, and sodium dihydrogen phosphate dihydrate such that their concentrations after reconstitution are 100 mg/mL, 2.2 mg/mL, and 6.84 mg/mL, respectively. The lyophilized preparation of the present invention may contain pentosan polysulfate sodium, disodium hydrogen phosphate dodecahydrate, and sodium dihydrogen phosphate dihydrate at a mass ratio of 800 to 1200 : 16 to 32 : 32 to 104, or at a mass ratio of 1000:22:68.4. Alternatively, the lyophilized preparation of the present invention may contain pentosan polysulfate sodium, disodium hydrogen phosphate, and sodium dihydrogen phosphate at a mass ratio of 800 to 1200 : 6 to 12 : 24 to 80, or at a mass ratio of 1000:8.72:52.6.

The lyophilized preparation of the present invention can be manufactured by lyophilizing an aqueous solution containing pentosan polysulfate sodium and additives such as a buffer according to claim 3.

Pentosan polysulfate sodium can be obtained by reacting xylan extracted from bark of a plant such as beech with a sulfating agent such as chlorosulfonic acid or chlorosulfuric acid to form a sulfate and treating the resultant sulfate using sodium hydroxide. Also, pentosan polysulfate sodium can be manufactured in consideration of U.S. Patent No. 2689848, U.S. Patent No. 5668116, and U.S. Publication No. 2009/0111771. Moreover, novel methods for manufacturing pentosan polysulfate sodium (WO2008/107906; WO2009/047699; WO2012/101544; WO2009/087581) are also reported, and pentosan polysulfate sodium may be manufactured in consideration of these methods.

For example, the lyophilized preparation of the present invention can be manufactured as a preparation that is stabler or is easy to reconstitute by lyophilizing an aqueous solution containing pentosan polysulfate sodium and a buffer at concentrations that are 0.25 to 0.75 times (preferably 0.5 times) as high as those after reconstitution. For example, the lyophilized preparation of the present invention can be manufactured by lyophilizing an aqueous solution containing pentosan polysulfate sodium at a concentration of 25 to 75 mg/mL (preferably 30 to 70, 40 to 60, or 50 mg/mL), disodium hydrogen phosphate dodecahydrate at a concentration of 0.55 to 1.65 mg/mL (preferably 0.6 to 1.6, 0.8 to 1.4, or 1.1 mg/mL), and sodium dihydrogen phosphate dihydrate at a concentration of 1.71 to 5.13 mg/mL (preferably 1.8 to 5.0, 2.0 to 4.0, or 3.42 mg/mL).

Acording to claim 2, the present invention encompasses such an aqueous solution to be used to manufacture a lyophilized preparation. Preferably, the aqueous solution to be used to manufacture the lyophilized preparation of the present invention is an aqueous solution containing pentosan polysulfate sodium and a buffer at concentrations that are 0.25 to 0.75 times (preferably 0.5 times) as high as those after reconstitution. The aqueous solution of the invention, which may be used to manufacture the lyophilized preparation of the present invention contains pentosan polysulfate sodium at a concentration of 25 to 75 mg/mL (preferably 30 to 70, 40 to 60, or 50 mg/mL), disodium hydrogen phosphate dodecahydrate at a concentration of 0.55 to 1.65 mg/mL (preferably 0.6 to 1.6, 0.8 to 1.4, or 1.1 mg/mL), and sodium dihydrogen phosphate dihydrate at a concentration of 1.71 to 5.13 mg/mL (preferably 1.8 to 5.0, 2.0 to 4.0, or 3.42 mg/mL).

Lyophilization can commonly include a preliminary freezing step, a primary drying step, and a secondary drying step. In the preliminary freezing step, freezing is commonly performed at a temperature that is lower than or equal to the eutectic point. For example, the preliminary freezing step can be performed at a constant temperature, but the temperature can also be changed as appropriate. In this specification, the preliminary freezing step can preferably include a step performed at -40 to -50°C for 30 minutes to 3 hours, a step performed at -10 to -20°C for 1 to 10 hours, and a step performed at -40 to -50°C for 30 minutes to 3 hours in the stated order.

The primary drying step is a step of sublimating frozen water under reduced pressure. It is known that water boils at 0°C under a pressure of 610.6 Pa, and a sublimation temperature decreases as the atmospheric pressure decreases. According to the present invention, the primary drying step is performed at -10°C to -20°C. In this specification, the primary drying step is preferably performed at 1 to 100 Pa and -10°C to -20°C, more preferably at 2 to 50 Pa and -12°C to -18°C, and most preferably at 10 Pa and -14°C. There is no particular limitation on the period of the primary drying step as long as all of the contained water can be sublimated, and the period can be set to 35 to 50 hours or 40 to 45 hours, for example. This may also be applied to a case of ten thousand 2.5-mL ampules.

The secondary drying step is a dehumidifying step for removing remaining water by increasing the temperature at lower pressure. In this specification, the secondary drying step is preferably performed by gradually increasing the temperature under a full vacuum environment. For example, the secondary drying step may be performed by increasing the temperature to 35 to 40°C under a full vacuum environment and then keeping the temperature at 35 to 40°C for 12 hours.

The sterilization degree or stability of the obtained lyophilized preparation can be maintained through capping, aluminum cap sealing, or the like, as needed.

This lyophilized preparation is excellent in stability required to supply the lyophilized preparation as a medicine. Specifically, the lyophilized preparation of this specification is stable at 40±2°C and 75%RH, which are known as the conditions of an acceleration test, for at least 6 months. In the medicine stability test, one month in an acceleration test is considered to correspond to 6 months of storage at ordinary temperatures and pressures, and therefore, this lyophilized preparation is stable at ordinary temperatures and pressures for 36 months. It is possible to confirm whether or not a lyophilized preparation to be tested is stable as described above by storing the lyophilized preparation at 40±2°C and 75% RH for 6 months and then analyzing whether or not the lyophilized preparation itself and a solution obtained by reconstituting the lyophilized preparation satisfy the specifications set for the preparation. When the preparation satisfies the specifications after the storage, it is determined that the preparation is stable under such storage conditions. Examples of the items of such specifications include the external appearance, the clarity and color, the average mass, the uniformity of mass, the uniformity of an administration unit, the mixing of minute particles (into the reconstituted solution): invisible minute particles, the mixing of minute particles (into the reconstituted solution): visible minute particles, the pH value (of the reconstituted solution), the drying loss, the transparency, the identification of a phosphate, the identification of pentosan polysulfate sodium (PPS) by gel permeation chromatography (GPC) and wet chemical analysis, the purity of sodium sulfate (IC), the purity (GPC), the purity of calcium, the PPS assay (GPC), the sterility, and the bacterial endotoxin. The following describes specific examples of the standards of these items: the external appearance: a white to bright yellow lyophilized product; the clarity and color: colorless to light yellow clear solution; the uniformity of mass: up to ±10% for 18 ampules, and up to ±20% for 2 ampules (average mass deviation); the acceptable value (AV) of the uniformity of an administration unit (Ph. Eur. 2.9.40): based on Ph. Eur.; the mixing of minute particles (into the reconstituted solution) (invisible minute particles) (Ph. Eur. 2.9.19): up to 6000 minute particles with a diameter of 10 pm or more per ampule, and up to 600 minute particles with a diameter of 25 pm or more per ampule; the mixing of minute particles (into the reconstituted solution) (visible minute particles) (Ph. Eur. 2.9.20): no minute particles contained; the pH value (of the reconstituted solution) (Ph. Eur. 2.2.3): 5.2 to 7.0; the identification of a phosphate (Ph. Eur. 2.3.1): Yes; the identification of PPS (GPC): retention time of a main peak in a chromatogram of a sample solution corresponds to retention time of a standard solution; the identification of PPS (wet chemical analysis): red to purple; the purity of sodium sulfate (IC): less than 3% when calculated from the applied PPS content; the purity (GPC): no additional peaks; the PPS assay (GPC): 95.0 to 105.0% when calculated from the applied PPS content; the sterility (Ph. Eur. 2.6.1): based on Ph. Eur.; and the bacterial endotoxin (Ph. Eur. 2.6.14): less than 300 IU/ml.

The lyophilized preparation of the present invention can be reconstituted by adding a sterile aqueous diluent, preferably sterile water, thereto and mixing them. The present invention includes a method for preparing a liquid pharmaceutical composition containing pentosan polysulfate sodium, the method including reconstituting the above-mentioned lyophilized preparation in a sterile aqueous diluent according to claim 7. The method for preparing a liquid pharmaceutical composition containing pentosan polysulfate sodium of the present invention includes adding a sterile aqueous diluent to a lyophilized preparation such that the concentration of pentosan polysulfate sodium is 80 to 120 mg/mL, and preferably 90 to 110 mg/mL, 95 to 105 mg/mL, or 100 mg/mL.

Also, the present invention encompasses a liquid pharmaceutical composition obtained through the reconstitution. In this specification, the terms "reconstituted solution", "reconstituted liquid pharmaceutical composition", and "liquid pharmaceutical composition obtained through reconstitution" are the same in meaning. It is more preferable that the liquid pharmaceutical composition contains pentosan polysulfate sodium, disodium hydrogen phosphate dodecahydrate, and sodium dihydrogen phosphate dihydrate such that their concentrations after reconstitution are 100 mg/mL, 2.2 mg/mL, and 6.84 mg/mL, respectively. Also, the pH of the liquid pharmaceutical composition is preferably 5.0 to 7.0.

The lyophilized preparation or reconstituted liquid pharmaceutical composition of the present invention can be used for humans and mammals such as oxen, deer, horses, donkeys, wild boars, pigs, sheep, goats, dogs, cats, raccoon dogs, foxes, rabbits, mice, and squirrels for medical purposes (treatment purposes or prevention purposes). For example, the lyophilized preparation of the present invention is for use as a therapeutic agent or preventive agent in the treatment or prevention of interstitial cystitis, osteoarthritis, lysosomal disease, and human lymphotropic virus type 1 (HTLV-1) associated myelopathy (HAM); or for use as an anticoagulant in a human or mammal. For example, U.S. Patent No. 9155784 discloses that pentosan polysulfate sodium has anti-TNFα activity and is effective at treating lysosomal disease. Lysosomal disease refers to a disease or disorder caused by the accumulation or presence of lysosomal enzymes due to abnormality in lysosomal enzymes. Examples of lysosomal disease include type-II glycogenosis, Pompe disease, α-glucosidase (acid maltase) deficiency, sphingolipidosis, GM1 gangliosidosis, GM2 gangliosidosis (Tay-Sachs disease, Sandhoff disease), metachromatic leukodystrophy (MLD), Fabry disease, Farber disease, Gaucher disease, Niemann-Pick disease (A, B, C types), Krabbe disease, mucopolysaccharidosis, mucolipidosis, multiple sulfatase deficiency (MSD), sialidosis, galactosialidosis, I-cell disease, α-mannosidosis, β-mannosidosis, fucosidosis, aspartylglucosaminuria, Schindler disease, Wolman disease, Danon disease, free sialic acid storage disease, and ceroid lipofuscinosis.

Also described herein is composition for use in a method of treating or preventing interstitial cystitis, osteoarthritis, lysosomal disease, or HAM, the method including administering an effective amount of a liquid pharmaceutical composition obtained by reconstituting the lyophilized preparation of the present invention to patients in need thereof. The term "patients in need thereof' means patients who are affected with or are likely to be affected with these diseases. Patients who are affected with these diseases and need to be treated are preferable. For example, when the above-mentioned liquid pharmaceutical composition is for use in therapeutic purposes or preventive purposes, the liquid pharmaceutical composition can be administered in the parenteral administration form such as an injection or infusion. The dose varies depending on the symptom, age, sex, weight, administration form, and the like, and when the composition is orally administered, for example, the daily dose per adult is commonly 100 to 1000 mg.

Hereinafter, the present invention will be described more specifically based on examples. However, the present invention is not limited to these examples.

### Examples

### Example 1: Manufacturing of Lyophilized Pentosan Polysulfate Sodium Preparation

First, 33.22 g of disodium hydrogen phosphate dodecahydrate was added to 13.00 kg of water for injection, and then 103.28 g of sodium dihydrogen phosphate dihydrate was added thereto. Next, 1574.6 g of pentosan polysulfate sodium was added to the resultant phosphate buffer and dissolved. Water for injection was added thereto, and then the pH was adjusted to 5.9 to 6.1 using 1 mol/L hydrochloric acid and 1 mol/L sodium hydroxide. Water for injection was added such that the total quantity was 31.05 kg and the resultant solution was stirred. The solution was sterilized using a 0.22-pm filter and was then filled into vials such that each vial contained 2.570 g of the solution.

The vials were half-capped with rubber stoppers, and then lyophilization was performed using a K1 lyophilizer (DFB-60R-07ASC) under the following conditions.

Preliminary freezing: reducing the temperature from room temperature to -42°C or lower (about -45°C); keeping the temperature at -42°C or lower (about -45°C) for 1 hour; increasing the temperature to -14°C; keeping the temperature at -14°C for 3 hours; reducing the temperature from -14°C to -42°C or lower (about -45°C); keeping the temperature at -42°C (about -45°C) for 1 hour

Primary drying: performed at a pressure of 10 Pa throughout, increasing the temperature from -42°C or lower (about -45°C) to -14°C over 1.5 hours; keeping the temperature at -14°C for 43 hours

Secondary drying: performed under reduced pressure (full vacuum condition) throughout, increasing the temperature from -14°C to 37°C; keeping the temperature at 37°C for 12 hours

After recovery of the pressure through N2 substitution, the vials were taken out and sealed with aluminum caps. Thus, the lyophilized pentosan polysulfate sodium preparation was obtained.

### Example 2: Stability Test on Lyophilized Pentosan Polysulfate Sodium Preparation

The lyophilized pentosan polysulfate sodium preparation manufactured using the method of Example 1 was stored at 40°C and 75%RH for 6 months, and then the stability thereof was evaluated (acceleration test).

Table 1 shows the results. It was shown that the lyophilized preparation met the standards of all of the evaluation items after 6-month storage. In particular, mixing of minute particles (visible minute particles) was never observed during the 6-month storage period, and thus it was also found that delamination did not occur.

**Table 1**

| Evaluation items | Standards | Initial analysis | 1 month after | 3 months after | 6 months after | Yes or No: meeting standards |
|---|---|---|---|---|---|---|
| External appearance | White to bright yellow lyophilized product | White lyophilized product | White lyophilized product | White lyophilized product | White lyophilized product | Yes |
| Clarity and color | Colorless to light yellow clear solution | Light yellow clear solution | Light yellow clear solution | Light yellow clear solution | Light yellow clear solution | Yes |
| Average mass | Reference | 130.5 mg | 130.1 mg | 130.6 mg | 132.7 mg | Yes |
| Uniformity of mass | Up to ±10% for 18 ampules, and up to ±20% for 2 ampules (average mass deviation) | Yes | Yes | Yes | Yes | Yes |
| Acceptable value (AV) of uniformity of administration unit (PhEur2940) | Based on Ph. Eur. | Yes | Yes | Yes | Yes | Yes |
| Mixing of minute particles (into reconstituted solution): invisible minute particles (Ph.Eur.2.9.19) | Up to 6000 minute particles with diameter of 10 µm or more per ampule, and up to 600 minute particles with diameter of 25 pm or more per ampule | 35 minute particles with diameter of 10 pm or more per ampule, and 4 minute particles with diameter of 25 pm or more per ampule | 97 minute particles with diameter of 10 pm or more per ampule, and 37 minute particles with diameter of 25 pm or more per ampule | 56 minute particles with diameter of 10 pm or more per ampule, and 15 minute particles with diameter of 25 pm or more per ampule | 35 minute particles with diameter of 10 pm or more per ampule, and 3 minute particles with diameter of 25 pm or more per ampule | Yes |
| Mixing of minute particles (into reconstituted solution): visible minute particles (Ph.Eur.2.9.20) | No particles | No particles | No particles | No particles | No particles | Yes |
| pH value (of the reconstituted solution) (Ph.Eur.2.2.3) | 5.2 to 7.0 | 5.9 | 5.9 | 5.9 | 6.0 | Yes |
| Drying loss (Ph.Eur.2.2.32) | Reference | 2.5% | 1.6% | 1.8% | 22% | Yes |
| Transparency | Reference | 78.4% | 79.3% | 79.1% | 690% | Yes |
| Identification of phosphate (Ph.Eur.2.3.1) | Yes | Yes | - | - | - | Yes |
| Identification of PPS (GPC) | Retention time of main peak in chromatogram of sample solution corresponds to retention time of standard solution | Yes | - | - | - | Yes |
| Identification of PPS (wet chemical analysis) | Red to purple | Yes | - | - | - | Yes |
| Purity of sodium sulfate (IC) | Less than 3% when calculated from applied PPS content | 0.9% | 1.0% | 0.9% | 1.1% | Yes |
| Purity (GPC) | No additional peaks | Yes | Yes | Yes | Yes | Yes |
| Purity of calcium | Reference | 0.1% | - | - | - | Yes |
| PPS assay (GPC) | 95.0 to 105.0% when calculated from applied PPS content | 101.3% | 1010% | 100.0% | 990% | Yes |
| Sterility (Ph.Eur.2.6.1) | Based on Ph. Eur. | Yes | - | - | Yes | Yes |
| Bacterial endotoxin (PhEur2614) | Less than 300 IU/ml | Yes | - | - | Yes | Yes |

## Claims

1. A lyophilized preparation for injection of pentosan polysulfate or a salt thereof, comprising pentosan polysulfate or a salt thereof, a buffer, and no cryoprotectants,
wherein the buffer is a phosphate buffer and comprises disodium hydrogen phosphate dodecahydrate in an amount such that the concentration is 1 to 4 mg/mL after reconstitution, and sodium dihydrogen phosphate dihydrate in an amount such that the concentration is 4.5 to 9 mg/mL after reconstitution, and
wherein the cryoprotectant is saccharide, and
wherein the salt of pentosan polysulfate is pentosan polysulfate sodium that is present in an amount such that the concentration is 80 to 120 mg/mL after reconstitution.

2. An aqueous solution for preparing a lyophilized pentosan polysulfate sodium preparation for injection according to claim 1, the aqueous solution comprising pentosan polysulfate sodium at a concentration of 25 to 75 mg/mL, disodium hydrogen phosphate dodecahydrate at a concentration of 0.55 to 1.65 mg/mL, and sodium dihydrogen phosphate dihydrate at a concentration of 1.71 to 5.13 mg/mL.

3. A method for manufacturing the lyophilized pentosane polysulfate sodium preparation for injection according to claim 1 , comprising performing lyophilization on the aqueous solution containing pentosan polysulfate sodium at a concentration of 25 to 75 mg/mL,
wherein the lyophilization includes a primary drying step performed at -10°C to -20°C.

4. The manufacturing method according to claim 3,
wherein the lyophilization includes a preliminary freezing step including:
keeping a temperature at -40 to -50°C for 30 minutes to 3 hours;
keeping the temperature at -10 to -20°C for 1 to 10 hours; and
keeping the temperature at -40 to -50°C for 30 minutes to 3 hours.

5. The lyophilized preparation for injection according to claim 1 for use as a therapeutic or preventive agent in the treatment or prevention of interstitial cystitis, osteoarthritis, lysosomal disease, or HAM (human lymphotropic virus type 1 (HTLV-1) associated myelopathy).

6. The lyophilized preparation for injection according to claim 1 for use as an anticoagulant in a human or mammal.

7. A method for preparing a liquid pharmaceutical composition comprising pentosan polysulfate or a salt thereof, the method comprising reconstituting the lyophilized preparation for injection according to claim 1 in a sterile aqueous diluent.

## Patentansprüche

1. Lyophilisiertes Präparat für die Injektion von Pentosanpolysulfat oder einem Salz davon, umfassend Pentosanpolysulfat oder ein Salz davon, einen Puffer und keine Kryoprotektoren,
wobei der Puffer ein Phosphatpuffer ist und Dinatriumhydrogenphosphatdodecahydrat in einer solchen Menge, dass die Konzentration nach der Rekonstitution 1 bis 4 mg/ml beträgt, und Natriumdihydrogenphosphatdihydrat in einer solchen Menge umfasst, dass die Konzentration nach der Rekonstitution 4,5 bis 9 mg/ml beträgt, und
wobei der Kryoprotektor ein Saccharid ist, und
wobei das Salz von Pentosanpolysulfat Pentosanpolysulfatnatrium ist, das in einer solchen Menge vorhanden ist, dass die Konzentration nach der Rekonstitution 80 bis 120 mg/ml beträgt.

2. Wässrige Lösung zum Herstellen eines lyophilisierten Pentosanpolysulfatnatriumpräparats für die Injektion nach Anspruch 1, wobei die wässrige Lösung Pentosanpolysulfatnatrium in einer Konzentration von 25 bis 75 mg/ml, Dinatriumhydrogenphosphatdodecahydrat in einer Konzentration von 0,55 bis 1,65 mg/ml und Natriumdihydrogenphosphatdihydrat in einer Konzentration von 1,71 bis 5,13 mg/ml umfasst.

3. Verfahren zum Herstellen des lyophilisierten Pentosanpolysulfatnatriumpräparats für die Injektion nach Anspruch 1, umfassend das Durchführen der Lyophilisierung der wässrigen Lösung, die Pentosanpolysulfatnatrium in einer Konzentration von 25 bis 75 mg/ml enthält,
wobei die Lyophilisierung einen primären Trocknungsschritt beinhaltet, der bei -10 °C bis - 20 °C durchgeführt wird.

4. Herstellungsverfahren nach Anspruch 3,
wobei die Lyophilisierung einen vorbereitenden Gefrierschritt beinhaltet, der beinhaltet:
Halten einer Temperatur bei -40 bis -50 °C für 30 Minuten bis 3 Stunden;
Halten der Temperatur bei -10 bis -20 °C für 1 bis 10 Stunden; und
Halten der Temperatur bei -40 bis -50 °C für 30 Minuten bis 3 Stunden.

5. Lyophilisiertes Präparat für die Injektion nach Anspruch 1 für die Verwendung als therapeutisches oder präventives Mittel bei der Behandlung oder Prävention von interstitieller Zystitis, Osteoarthritis, lysosomaler Krankheit oder HAM (mit humanem lymphotropem Virus, Typ 1 (HTLV-1-) assoziierte Myelopathie).

6. Lyophilisiertes Präparat für die Injektion nach Anspruch 1 für die Verwendung als ein Antikoagulans bei einem Menschen oder einem Säugetier.

7. Verfahren zum Herstellen einer flüssigen pharmazeutischen Zusammensetzung, umfassend Pentosanpolysulfat oder ein Salz davon, wobei das Verfahren das Rekonstituieren des lyophilisierten Präparats für die Injektion nach Anspruch 1 in einem sterilen wässrigen Verdünnungsmittel umfasst.

## Revendications

1. Préparation lyophilisée pour injection de pentosane polysulfate ou un sel de celui-ci, comprenant du pentosane polysulfate ou d'un sel de celui-ci, un tampon et aucun cryoprotecteur, dans laquelle le tampon est un tampon phosphate et comprend de l'hydrogénophosphate disodique dodécahydraté en une quantité telle que la concentration est de 1 à 4 mg/mL après reconstitution, et du dihydrogénophosphate de sodium dihydraté en une quantité telle que la concentration est de 4,5 à 9 mg/mL après reconstitution, et
dans lequel le cryoprotecteur est un saccharide, et
dans lequel le sel de pentosane polysulfate est le pentosane polysulfate de sodium présent en une quantité telle que la concentration soit de 80 à 120 mg/mL après reconstitution.

2. Solution aqueuse pour préparer une préparation pentosane lyophilisé de polysulfate de sodium pour injection selon la revendication 1, la solution aqueuse comprenant du pentosane polysulfate de sodium à une concentration de 25 à 75 mg/mL, du phosphate disodique dodécahydraté à une concentration de 0,55 à 1,65 mg/mL et du dihydrogénophosphate de sodium dihydraté à une concentration de 1,71 à 5,13 mg/mL.

3. Procédé de fabrication de la préparation de pentosane lyophilisé injectable de polysulfate de sodium selon la revendication 1, comprenant la réalisation d'une lyophilisation sur la solution aqueuse contenant du pentosane polysulfate de sodium à une concentration de 25 à 75 mg/mL, dans lequel la lyophilisation comprend une étape de séchage primaire réalisée entre -10°C et - 20°C.

4. Procédé de fabrication selon la revendication 3,
dans lequel la lyophilisation comprend une étape préliminaire de congélation comprenant les étapes consistant à :
maintenir une température entre -40 et -50°C pendant 30 minutes à 3 heures ;
maintenir la température entre -10 et -20°C pendant 1 à 10 heures ; et
maintenir la température entre -40 et -50°C pendant 30 minutes à 3 heures.

5. Préparation lyophilisée pour injection selon la revendication 1 destinée à être utilisée comme agent thérapeutique ou préventif dans le traitement ou la prévention de la cystite interstitielle, de l'arthrose, de la maladie lysosomale ou de la HAM (myélopathie associée au virus lymphotrope humain de type 1 (HTLV-1)).

6. Préparation lyophilisée pour injection selon la revendication 1, destinée à être utilisée comme anticoagulant chez un être humain ou un mammifère.

7. Procédé de préparation d'une composition pharmaceutique liquide comprenant du pentosane polysulfate ou un sel de celui-ci, le procédé comprenant la reconstitution de la préparation lyophilisée pour injection selon la revendication 1 dans un diluant aqueux stérile.
